(19) 

Europäisches
Patentamt
European
Patent Office
Office européen
des brevets

(11) **EP 3 647 432 A1**

(12) **EUROPEAN PATENT APPLICATION**

(43) Date of publication:
**06.05.2020 Bulletin 2020/19**

(21) Application number: **18204120.2**

(22) Date of filing: **02.11.2018**

(51) Int Cl.:
*C12P 23/00* (2006.01)  *B01D 11/00* (2006.01)
*G01N 30/42* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Technische Universität München 80333 München (DE)**

(72) Inventors:
• **MINCEVA, Mirjana**
 **85354 Freising (DE)**
• **BAUER, Andreas**
 **85354 Freising (DE)**

(74) Representative: **Engelhard, Markus**
 **Boehmert & Boehmert**
 **Anwaltspartnerschaft mbB**
 **Pettenkoferstrasse 22**
 **80336 München (DE)**

(54) **METHOD OF EXTRACTING A PIGMENT FROM MICROALGAE**

(57) The present invention relates to a method of extracting a pigment from microalgae.

EP 3 647 432 A1

**Description**

**FIELD OF THE INVENTION**

The present invention relates to a method of extracting a pigment from microalgae.

**BACKGROUND OF THE INVENTION**

Astaxanthin ($C_{40}H_{52}O_4$), also referred to as 3,3'-dihydroxy-β,β'-carotene-4,4'-dione, is a red carotenoid which is used as colorant in the food industry, for example in the aquaculture of salmon, as well as in the cosmetic industry. Astaxanthin is a strong antioxidant, stabilizing and reducing free radicals. Astaxanthin naturally occurs in the microalgae *Haematococcus pluvialis (H. pluvialis).* Astaxanthin can be extracted from *H. pluvialis,* however, the extraction is accompanied by high costs, such as for purification of the product.

Additionally, astaxanthin can be produced synthetically. However, synthetic astaxanthin has a 20-fold lower antioxidant effect than biotechnologically produced astaxanthin from *H. pluvialis* [1].

Biotechnological production of astaxanthin using *H. pluvialis* is performed by enrichment of astaxanthin in said microalgae, e.g. by nitrate depletion or high light intensity, which results in accumulation of astaxanthin in the cytoplasm of a *H. pluvialis* cell to up to 5 wt.-% dry weight. However, astaxanthin synthesis is accompanied by formation of a thick, resistant cell wall, which renders direct extraction of astaxanthin from said cells having a thick cell wall to be an inefficient process. During biotechnological production of astaxanthin, the cell suspension is commonly concentrated by centrifugation and subsequently dried. After mechanical disruption of the cells using a bead mill, the extraction of astaxanthin from the biomass is performed using supercritical carbon dioxide which has to be compressed up to 1000 bar [2]. Extraction using carbon dioxide is a common astaxanthin-extraction process, however, the process is very energy-consuming due to the drying step and the high pressures needed for supercritical carbon dioxide. Additionally, each process step is accompanied by astaxanthin yield loss, which negatively influences the total efficiency of the process.

Due to said thick cell wall in the cyst stage of *H. pluvialis,* a direct extraction of astaxanthin in a solvent is not possible. An approach for natural disruption of said thick cell wall is inducing germination by means of subjecting said cell in the cyst stage to vegetative growth conditions. Such germination-inducing conditions induce germination of *H. pluvialis* in the cyst stage to enter a flagellated stage [3]. In said flagellated stage, the cells do not have a thick cell wall and do only have a thin cell membrane for a short period of time. Praveenkumar et al. 2014 have described the extraction of astaxanthin from cells in said flagellated stage by centrifugation of cells in their flagellated stage and extraction of astaxanthin from said cells using an ionic liquid [3]. However, ionic liquids are very costly and thus not suitable for industrial scale extraction of astaxanthin.

Centrifugal partition chromatography (CPC) have been used for extracting carotenoids from algae and yeast. For example, Marchal et al. used a CPC system to extract β-carotene from *Dunaliella salina (D. salina)* using a biocompatible solvent [4]. In contrast to *H. pluvialis,* which contains a thick cell wall in the cyst stage, *D. salina* does not contain a thick cell wall in any cell stage, which allows for a direct extraction of a pigment from *D. salina* using a solvent.

An advantage of CPC systems, as well as of countercurrent chromatography (CCC) systems, is that mechanical stress and disruption of cells during the extraction process can be reduced by optimizing operational parameters. Therefore, cells that have undergone a method of extraction using such an optimized process can be re-cultured and used in multiple extractions [4].

Du et al. [5] used a counter-current chromatography (CCC) system for isolation and purification of astaxanthin from a *Phaffia rhodozyma* extract. The astaxanthin-rich extract was obtained after disruption of the cells with DSMO, followed by several extraction steps using different solvents. The extract was injected in to a CCC unit and separated using a biphasic system composed of n-hexane-acetone-ethanol-water (1:1:1:1, v/v/v/v) resulting in a yield of 20.6 mg astaxanthin at 92.0% purity from 100 mg crude extract of *Phaffia rhodozyma*.

However, there is a need of a method for extraction of a pigment which is efficient with regard to yield and solvent consumption, preferably allowing for reduced solvent consumption and reduced cost for solvent recovery.

The aim of the present invention is an efficient method of extracting a pigment from microalgae, particularly astaxanthin from *H. pluvialis.* A further aim is to optimize astaxanthin extraction from microalgae by replacing energy-intensive process steps such as concentration of the biomass, drying, and extracting astaxanthin with supercritical carbon dioxide, with an efficient process step allowing to increase efficiency and to reduce costs. A further aim is to make a pigment such as astaxanthin, which is enriched in a microalga, accessible to directly extracting said pigment using a solvent.

**SUMMARY OF THE INVENTION**

The technical problem is solved by providing a method of extracting a pigment from microalgae, comprising the following steps: providing microalgae in an aqueous culture medium, wherein said microalgae are enriched with a pigment, inducing said microalgae to enter a flagellated stage using a germination-inducing condition, and/or disrupting said microalgae resulting in a suspension comprising said disrupted microalgae and said pigment, and extracting said pigment from said flagellated microalgae and/or from said suspension using a liquid-liquid extraction system comprising a solvent.

In one embodiment, a method of the present invention uses the properties of the natural cell cycle of a microalga for direct extraction of a pigment such as astaxanthin with a solvent. In one embodiment, the method of the present invention differs from conventional astaxanthin production in that the pigment is not directly extracted from said cyst cells after stress-induced astaxanthin production, in contrast, flagellated cell stages, in which cells have only a thin cell membrane, are induced prior to extraction of said pigment. In one embodiment, germination of said cyst cells is induced prior to or after concentrating said cells by centrifugation. In one embodiment, said flagellated cells are optionally subjected to mechanical disruption before said pigment is extracted.

The present invention allows for direct extraction of astaxanthin from microalgae in their flagellated stage using a liquid-liquid extraction system. Thereby, costly processes such as drying, dehydration, and extraction using supercritical carbon dioxide, are avoided, and the efficiency of astaxanthin extraction is enhanced.

Additionally, besides being cost- and time-efficient, another advantage of a method of the present invention is that, in one embodiment using long chained solvents such as decane or dodecane, it can be carried out non-invasively with regard to said microalgae cells, so that astaxanthin can be extracted from microalgae and subsequently said microalgae can be re-cultured to enrich astaxanthin which subsequently can be extracted after enrichment within the microalgae. Thus, in one embodiment, a method of the present invention can be performed multiple times with the same stock of microalgae.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The present invention relates to method of extracting a pigment from microalgae, comprising the following steps:

a) Providing microalgae in an aqueous culture medium, wherein said microalgae are enriched with a pigment,
b) Inducing said microalgae to enter a flagellated stage using a germination-inducing condition, and/or disrupting said microalgae resulting in a suspension comprising said disrupted microalgae and said pigment,
c) Extracting said pigment from said flagellated microalgae and/or from said suspension using a liquid-liquid extraction system comprising a solvent, wherein the liquid-liquid extraction system is selected from a countercurrent chromatography system, a centrifugal partition chromatography system, and a membrane-assisted liquid-liquid extraction system.

In one embodiment, said method comprises the following steps:

a) Providing microalgae in an aqueous culture medium, wherein said microalgae are enriched with a pigment,
b) Inducing said microalgae to enter a flagellated stage using a germination-inducing condition,
c) Extracting said pigment from said flagellated microalgae using a liquid-liquid extraction system comprising a solvent, wherein the liquid-liquid extraction system is selected from a countercurrent chromatography system, a centrifugal partition chromatography system, and a membrane-assisted liquid-liquid extraction system.

In one embodiment, said method comprises the following steps:

a) Providing microalgae in an aqueous culture medium, wherein said microalgae are enriched with a pigment,
b) Disrupting said microalgae resulting in a suspension of said disrupted microalgae and said pigment,
c) Extracting said pigment from said suspension using a liquid-liquid extraction system comprising a solvent, wherein the liquid-liquid extraction system is selected from a countercurrent chromatography system, a centrifugal partition chromatography system, and a membrane-assisted liquid-liquid extraction system.

In one embodiment, said microalgae are initially in a cyst stage and are induced to enter a flagellated stage by means of a germination-inducing condition.
In one embodiment, said germination-inducing condition is selected from a phototrophic condition, a mixotrophic condition, and a heterotrophic condition.

In one embodiment, wherein said microalgae are disrupted mechanically, for example by using a homogenizer, a grinder, a bead mill, beadbeating, a blender, sonication, pressure cycling, a microfluidizer, an expeller press, or freezing and thawing cycles. In one embodiment, said step c) is followed by step d) obtaining said pigment by lyophilization, freezing, vaporization of said solvent, or by dissolving said pigment in a nutritional oil, or another solvent, such as an organic solvent, a water based solution, a plant oil, or a deep eutectic solvent, or a combination thereof.

In one embodiment, said microalgae have become enriched with said pigment by nutrient depletion, excessive light exposure, high salinity, and/or overexpression resulting from genetic modification of said microalgae.

In one embodiment, said microalgae are Chlorophyta, preferably Chlorophyceae, more preferably *Haematococcus pluvialis.*

In one embodiment, said microalgae are selected from *Haematococcus pluvialis, Chlorella zofingiensis, Neochloris wimmeri,* and *Chlamydomonas nivalis.*

In one embodiment, said liquid-liquid extraction system is a membrane-assisted liquid-liquid extraction system.

In one embodiment, said liquid-liquid extraction system is a liquid-liquid chromatography system selected from a centrifugal partition chromatography system and a countercurrent chromatography system.

In one embodiment, said solvent has a vapor pressure of at least 10 mbar, preferably of at least 64 mbar, at 25 °C and ambient pressure.

In one embodiment, said solvent is selected from methyl-tert-butyl ether, ethyl acetate, butan-1-ol, dichloromethane, chloroform, diethyl ether, ethyl methyl ether, toluene, benzene, ketone, 1,1-dichloroethane, cyclohexane, isopropyl acetate, 2-methyltetrahydofuran, methyl ethyl ketone, methylcyclohexane, 2,2,4-trimethylpentane, xylene, pentan-1-ol, dodecane, decane, acetone, ethanol, propan-2-ol, propan-1-ol, methanol, tetrahydrofuran, tert-butanol, acetonitrile, dimethyl sulfoxide, acetic acid, ethylene glycol, n-alkanes, and oil such as nutritional oil, or a combination thereof, preferably selected from ethyl acetate and methyl-tert-butyl ether, or a combination thereof.

In one embodiment, said pigment is a keto-carotenoid, preferably astaxanthin.

In one embodiment, said method comprises the following steps:

a) Providing microalgae in an aqueous culture medium, wherein said microalgae are enriched with a pigment,
b) Inducing said microalgae to enter a flagellated stage using a germination-inducing condition, and optionally disrupting said microalgae resulting in a suspension comprising said disrupted microalgae and said pigment,
c) Extracting said pigment from said flagellated microalgae and/or from said suspension using a liquid-liquid extraction system comprising a solvent, wherein the liquid-liquid extraction system is selected from a countercurrent chromatography system, a centrifugal partition chromatography system, and a membrane-assisted liquid-liquid extraction system.

## DETAILED DESCRIPTION

The term "providing microalgae in an aqueous culture medium", as used herein, relates to providing microalgae for extraction of a pigment. In one embodiment, said providing relates to providing microalgae in a cyst stage. Said microalgae in a cyst stage may be induced to enter a flagellated stage to allow for direct extraction of a pigment from said flagellated stage and thus non-disruptive extraction from said cells. Alternatively, in one embodiment, said providing may relate to providing microalgae in a cyst stage, wherein said microalgae in a cyst stage are disrupted prior to extraction of said pigment and said pigment can be extracted from the resulting suspension of disrupted cells. In one embodiment, said providing relates to providing microalgae in a flagellated stage. In one embodiment, said flagellated cells can be used for direct extraction of a pigment and thus non-disruptive extraction, and/or said flagellated cells can be disrupted and said pigment can be extracted from the resulting suspension of disrupted cells. In one embodiment, said providing relates to providing a mixed culture of microalgae containing both microalgae in a cyst stage and in a flagellated stage, wherein said microalgae in a cyst stage or in a flagellated stage each can be intact or disrupted.

The term "extracting", as used herein, relates to a separation process in which a substance, such as a pigment, is separated from a substance mixture, such as cell culture medium containing cells enriched with a pigment using a solvent. In order to allow for an efficient extraction of a pigment, the solvent should be able to dissolve the pigment selectively and to dissolve a large amount of pigment. If a suitable solvent is used as extraction means, the substance to be extracted dissolves better in the solvent than in the substance mixture, and the solvent thus extracts the substance out of the substance mixture.

The term "pigment", as used herein, relates to a material that changes the color of reflected or transmitted light as the result of wavelength-selective absorption. In one embodiment, a pigment refers to a substance selected from primary carotenoids such as violaxanthin, neoxanthin, lutein, zeaxanthin, and $\beta$-carotene, other carotenoids such as adonixanthin, adonirubin, canthaxanthin, and echinenone, astaxanthin in its free form, and astaxanthin in the form of mono- or diesters with fatty acids. In one embodiment, a pigment refers to a xanthophyll or a keto-carotenoid. In one preferred embodiment, a pigment relates to astaxanthin. In one embodiment, a pigment may be present in a free form, or in derivate form, such as a fatty acid ester of a pigment.

The term "enriched with a pigment", as used herein, relates to accumulation of pigments within microalgae cells. Said microalgae can become enriched with a pigment such as astaxanthin by, for example, nutrient depletion, particularly nitrate and/or phosphate depletion, excessive light exposure, high salinity, and/or metabolic engineering. In one embodiment, microalgae are genetically engineered to overexpress proteins that are involved in pigment production. In one embodiment, overexpression of proteins involved in pigment production resulting from genetic modification, i.e. metabolic engineering, of said microalgae leads to overproduction of pigments within said engineered microalgae. In one embodiment, a microalga selected from *Haematococcus pluvialis, Chlorella zofingiensis, Neochloris wimmeri,* and *Chlamydomonas nivalis* is enriched with a pigment, preferably with astaxanthin. In one embodiment, cyanobacteria are enriched with a pigment.

The term "excessive light exposure", as used herein, relates to subjecting microalgae to higher intensity of light and/or other wavelengths of light than said microalgae are subjected to under normal, physiological conditions, wherein said subjecting results in the induction of a stressed state in said microalgae.

The term "high salinity", as used herein, relates to subjecting microalgae to higher concentrations of salt and/or other types of salts than said microalgae are subjected to under normal, physiological conditions, wherein said subjecting results in the induction of a stressed state in said microalgae.

The term "stressed state", as used herein, relates to a dormant phase of a microalga in which said microalga forms aplanospores (cysts). In a stressed state, a microalga becomes self-protective by forming a thick cell wall and may accumulate high levels of secondary carotenoids such as astaxanthin. In one embodiment, a stressed state is induced by non-vegetative growth conditions such as under excessive light exposure, high salinity, and/or nutrient depletion.

The term "keto-carotenoid", as used herein, relates to a carotenoid which contains a ketone group, wherein a carotenoid is an organic pigment belonging to the tetraterpenoids.

The term "xanthophyll", as used herein, relates to yellow pigments that form one of two major divisions of the carotenoid group. Xanthophylls are structurally similar to carotenes except containing oxygen atoms. Xanthophylls comprise substances such as astaxanthin, zeaxanthin, and neoxanthin.

The term "astaxanthin", as used herein, relates to a keto-carotenoid that belongs to the class of terpenes. Astaxanthin is a lipid-soluble pigment and can be used as dietary supplement. It exhibits a red-orange color which derives from the extended chain of conjugated double bonds at the center of the compound. Astaxanthin naturally occurs in microalgae, yeast, salmon, trout, krill, shrimp, crayfish, crustaceans, and the feathers of some birds, and may further occur in genetically modified organisms, such as Escherichia coli bacteria.

The term "microalgae", as used herein, relates to microscopic algae which are unicellular species which exist individually, in chains, or in groups. Typically, microalgae are found in freshwater systems or marine systems. Microalgae may produce products such as carotenoids, antioxidants, fatty acids, enzymes, polymers, peptides, toxins, and sterols. In one embodiment, a microalga relates to *Chlorella zofingiensis, Neochloris wimmeri, or Chlamydomonas nivalis.* In one embodiment, the term "microalgae" may also include cyanobacteria. In one preferred embodiment, a microalga relates to *Haematococcus pluvialis.* In one embodiment, the terms "microalga" and "cell" are used interchangeably. The term "culture medium", as used herein, relates to a medium which is used to cultivate and nourish a microalga, for example *H. pluvialis.* Such a medium may contain nutrients such as nitrogen, phosphorus, potassium, sulfur, iron, magnesium, sodium, calcium, chlorine, zinc, copper, boron, molybdenum, manganese, nickel, vanadium, vitamin B12, biotin, and thiamine. In one embodiment, a suitable culture medium is selected from Bold Modified Basal Freshwater Nutrient Solution (BBM), BG-11 medium, OHM medium, and KM1-medium. In one embodiment, said culture medium may contain one or more of the mentioned nutrients in a certain combination. Additionally a carbon source, such as glucose, fructose, acetate, glycerol, glutamate, lactate, alanine, aspartic acid, glutamine, or acetic acid can be added.

The term "cyst stage", as used herein, relates to a dormant stage of a microorganism, such as a microalga. Encystment of a microorganism allows for withstanding conditions in an unfavorable environment, such as lack of nutrients or oxygen, extreme temperatures, lack of moisture and presence of toxic chemicals. Microorganisms such as *H. pluvialis* may have a rigid cell wall structure in their cyst stage, consisting of several layers including the trilaminar sheath (TLS), secondary wall (SW), and tertiary wall (TW).

The term "flagellated stage", as used herein, relates to a stage of a microalga, in which said microalga is characterized by having one or more flagellae. In one embodiment, a microalga in its flagellated stage is motile. In one embodiment, a microalga in its flagellated stage does not have a thick cell wall. In one embodiment, a microalga in a cyst stage can be induced to enter a flagellated stage by germination-inducing conditions, resulting in the loss of the thick cell wall present in the cyst stage. In one embodiment, pigments enriched in a microalga cell in a cyst stage are retained in said cell, which is initially in a cyst stage and then enters a flagellated stage, until said pigment is extracted from said cell directly or from said cell in disrupted form. The flagellated stage of *H. pluvialis* does not comprise the rigid cell wall structure of cyst stage *H. pluvialis. H. pluvialis* in the flagellated stage are surrounded by a thin cell membrane. In one embodiment, *H. pluvialis* cells which are induced to enter the flagellated stage retain the astaxanthin that enriched within said cells during being in a cyst stage. In one embodiment of the present invention, the lack of a thick cell wall in the flagellated stage of *H. pluvialis* enables using said cells without a thick cell wall for efficient astaxanthin extraction method. In one embodiment, intracellular astaxanthin is directly extracted from a microalga in flagellated stage into a solvent without mechanical disruption of said microalga.

The term "germination-inducing condition", as used herein, relates to a condition that induces germination in a microorganism such as a microalga. In one embodiment, the temperature range in a "germination-inducing condition" according to the present invention is between 10 °C and 35 °C, preferably between 20 °C and 30 °C. In one embodiment, "germination-inducing condition" and "vegetative growth condition" are used synonymously. A condition which induces germination in a microalga, such as *Haematococcus pluvialis,* may be selected from, for example, phototrophic, mixotrophic, and heterotrophic conditions. In one embodiment, said condition comprises one or more components selected from nitrogen, phosphorus, potassium, sulfur, iron, magnesium, sodium, calcium, chlorine, zinc, copper, boron, molybdenum, manganese, nickel, vanadium, vitamin B12, biotin, and thiamine. In one embodiment, said germination-inducing condition is a phototrophic germinating condition and comprises a $CO_2$ content of 0.01 to 10 % v/v, an aeration of 0.01 to 5 wm, and light. In one embodiment, light in the wavelength range from 300 nm to 800 nm is used, preferably with larger quantities in the range from 400 nm to 500 nm and/or 550 nm to 700 nm. The light intensity (photon flux density) is typically in a range of 10 to 10000 $\mu$mol m$^{-2}$s$^{-1}$.

In one embodiment, said germination-inducing condition is a mixotrophic germinating condition and comprises a $CO_2$ content of 0.01 to 10 % v/v, an aeration of 0.01 to 5 vvm, light, and a carbon source such as acetate, acetic acid, glycerol, glutamate, lactate, alanine, aspartic acid, glutamine, and/or sugars such as glucose and fructose. In one embodiment, light in the wavelength range from 300 nm to 800 nm is used, preferably with larger quantities in the range from 400 nm to 500 nm and/or 550 nm to 700 nm. The light intensity (photon flux density) is typically in a range of 10 to 10000 $\mu$mol m$^{-2}$s$^{-1}$.

In one embodiment, said germination-inducing condition is a heterotrophic condition and comprises a carbon source such as acetate, acetic acid, glycerol, glutamate, lactate, alanine, aspartic acid, glutamine, and/or sugars such as glucose and fructose, as well as aeration with 0.01 to 5 vvm air.

The term "suspension", as used herein, relates to a heterogeneous mixture that contains solid particles and that may also contain solutes. In one embodiment, a suspension contains a liquid and microalgae in their cyst stage and/or their flagellated stage. In one embodiment, a suspension contains a liquid and disrupted microalgae, wherein said disrupted microalgae may derive from microalgae cells in a cyst stage or a flagellated stage. In one embodiment, a suspension contains a liquid and microalgae in their cyst stage and/or their flagellated stage, and contains disrupted microalgae. In one embodiment, the term "cell suspension" is used synonymously with the terms "fermentation broth" or "cell broth".

The terms "fermentation broth" or "cell broth", as used herein, relates to culture medium, or a different aqueous medium, which contains microalgae cells in their flagellated stage, and/or in their cyst stage, and/or disrupted cells, wherein said disrupted cells are disrupted flagellated cells, and/or disrupted cyst cells. In one embodiment, fermentation broth, cell broth, and algae broth are used interchangeably.

The term "disrupting said microalgae", as used herein, relates to a process of disrupting the cell integrity of microalgae, wherein the resulting cells are referred to as "disrupted cells". By disrupting a microalga, the components of the cell interior of said microalga get released, such as pigments stored within said cell, for example astaxanthin. Disrupting microalgae can be performed, for example, by mechanically disrupting said cells, for example using a homogenizer, a grinder, a bead mill, beadbeating, a blender, sonication, pressure cycling, a microfluidizer, an expeller press, or freezing and thawing cycles. "Disrupted cells" are cells deriving from cells in a cyst stage or in a flagellated stage, which are disrupted.

The term "liquid-liquid chromatography system", as used herein, relates to a chromatography system which employs a liquid mobile phase and a liquid stationary phase. In one embodiment, said liquid-liquid chromatography system relates to a chromatography system which employs a liquid mobile phase and a liquid stationary phase that is kept in the system with the help of a centrifugal field. In liquid-liquid chromatography, the separation of substances of a mixture results from the distribution of the substances between the two immiscible liquid phases. In one embodiment, a liquid-liquid chromatography system relates to a countercurrent chromatography system (CCC) or a centrifugal partition chromatography system (CPC).

The term, "centrifugal partition chromatography" or "CPC", as used herein, relates to a chromatographic technique in which stationary and mobile phase are liquid, and the stationary phase is immobilized by the application of a centrifugal field. A centrifugal partition chromatography system comprises a connected network of extraction chambers. Annular disks and annular plates are the core of a CPC system. Chambers are milled into the annular disks, which are connected with each other by means of channels. Between each annular disk there is an annular plate which connects the last chamber of an annular disk with the next one through a hole. The annular disk and annular plate are alternately placed on top of each other and mounted on the axis of a centrifuge. A centrifugal force is generated by rotation, which is why one phase is retained in the chambers (stationary phase), while another phase (mobile phase) is pumped from chamber to chamber. The mobile phase is pumped from chamber to chamber and flows through the stationary phase towards the centrifugal field if it is the denser phase (this mode is called descending mode), or in centripetal direction if the mobile phase is the less dense phase (ascending mode). A commercially available CPC system can be used to carry out a method of extracting a pigment according to the present invention.

The organic solvent is held stationary in the chambers of a CPC system by applying a centrifugal field. A CPC system allows for enhancing the efficiency of an extraction by means of centrifugation, since a dispersion of the aqueous phase, such as a cell suspension, into the organic phase is enhanced. A suitable solvent has to be identified that has a low solubility in water, that can be stationary hold in a CPC system, and that is able to extract astaxanthin from microalgae in a flagellated stage, or from disrupted microalgae in a cyst stage or a flagellated stage, or both. After extracting astaxanthin and saturation of said solvent, said astaxanthin-containing solvent is replaced within the CPC system by water that is pumped into said CPC system, and said astaxanthin-containing solvent is thus obtained. In one embodiment, the obtained astaxanthin-containing solvent is treated by vaporization of said solvent, so that concentrated astaxanthin is obtained. Thereby, the method of the present invention is a very efficient process which allows to modify and/or replace energy- and time-consuming process steps in the astaxanthin-extraction, such as drying of biomass, mechanical cell disruption, and carbon dioxide extraction.

The term "countercurrent chromatography " or "CCC", as used herein, relates to a liquid-liquid chromatographic technique in which mobile and stationary phases are liquid, and the stationary phase is immobilized with the application of centrifugal field. A CCC column is an open tube coiled on spools that rotate in a planetary motion. The planetary motion in the CCC spools changes the intensity and direction of the centrifugal field. At high centrifugal fields, phase decantation (settling) occurs while at a reversion of the centrifugal field the two phases mix and allow a mass transfer/extraction of the target compound. The mixing and settling zones are successively distributed along the whole tube length. Similar to CPC, the CCC unit can operate in ascending mode (mobile phase is the less dense phase) or descending mode (mobile phase is the denser phase). A commercially available CCC system can be used to carry out a method of extracting a pigment according to the present invention.

The organic solvent is held stationary in the coiled tube of a CCC system with the application centrifugal field. A CCC system allows for enhancing the efficiency of an extraction by means of centrifugation, since a dispersion of the aqueous phase, such as a cell suspension, into the organic phase is enhanced. A suitable solvent has to be identified that has a low solubility in water, that can be stationary hold in a CCC system, and that is able to extract astaxanthin from microalgae in a flagellated stage, or from disrupted microalgae in a cyst stage or a flagellated stage, or both. After extracting astaxanthin and saturation of said solvent, said astaxanthin-containing solvent is replaced within the CCC system by water that is pumped into said CCC system, and said astaxanthin-containing solvent is thus obtained. In one embodiment, the obtained astaxanthin-containing solvent is treated by vaporization of said solvent, so that concentrated astaxanthin is obtained. Thereby, the method of the present invention is a very efficient process which allows to modify and/or replace energy- and time-consuming process steps in the astaxanthin-extraction, such as drying of biomass, mechanical cell disruption, and carbon dioxide extraction.

The term "liquid-liquid extraction system", as used herein, relates to a means to extract a pigment from microalgae, wherein said extraction is performed using two liquid phases. In one embodiment, said liquid-liquid extraction system relates to a system selected from a liquid-liquid chromatography system, such as centrifugal partition chromatography and countercurrent chromatography, and a membrane-assisted liquid-liquid extraction system. In one embodiment, said liquid-liquid extraction system is selected from centrifugal partition chromatography, countercurrent chromatography, and membrane-assisted liquid-liquid extraction.

The term, "membrane-assisted liquid-liquid extraction", as used herein, relates to a method of extracting a compound such as a pigment which involves two liquid phases separated by a membrane. A liquid-liquid interface is formed at each pore mouth of said membrane and the membrane serves as a physical separation barrier between the feed (fermentation broth) and extracting phase (solvent or mixture of solvents). The liquid-liquid interface is stabilized by a slight overpressure on one side of the membrane. In one embodiment, membrane-assisted liquid-liquid extraction comprises using a hollow fiber contactor to allow a non-dispersive contact of two liquid phases via a microporous membrane. In one embodiment, said two liquids are an organic solvent phase and an algae fermentation broth, and a pigment comprised in the fermentation broth will be distributed between the phases according to its partition coefficient. In one embodiment, using a hollow fiber contactor for extraction has several advantages over conventional liquid-liquid extraction units such as mixer-settler systems, namely that no dispersion of one phase in the other phase is necessary and no separation of the two phases is needed after extraction, no danger of formation of a stable emulsion is present, the two liquid phases may have the same densities, the two phases may have different temperatures, and that there are large specific exchange surfaces. In one embodiment, said membrane-assisted liquid-liquid extraction relates to pertraction and/or membrane-supported extraction. In one embodiment, polymers typically used for an extraction system are polypropylene (PP), polyvinylidene fluoride (PVDF), and/or polytetrafluoroethylene (PTFE).

The term "nitrate", as used herein, relates to a polyatomic ion with the molecular formula $NO_3$, $NH_4^+$, urea, and salts or derivatives thereof. The term also comprises salts of said polyatomic ion, such as inorganic nitrate salts including potassium nitrate, ammonium nitrate, sodium nitrate, calcium nitrate, magnesium nitrate. The term also relates to other forms of nitrate, such as in form of amino groups from amino acids, e.g. asparagine.

The term "phosphate", as used herein, relates to phosphate ion, i.e. $PO_4^{3-}$, and salts or derivatives thereof. The term also relates to inorganic phosphate salts, such as sodium phosphate, calcium phosphate, potassium phosphate, rubidium phosphate, and ammonium phosphate.

The term "obtaining pigment", as used herein, relates to any process that allows to obtain a pigment of interest in a suitable form for storage, transportation, and/or commercial and/or scientific distribution. Such process may, for example, be lyophilizing said pigment, drying said pigment, freezing said pigment, dissolving said pigment in a solvent such as an organic solvent, a water-based solution, a plant oil, a deep eutectic solvent, or a mixture thereof, and dissolving said pigment in a pharmaceutically or nutritionally acceptable solvent such as nutritional oil. In one embodiment, said obtaining relates to obtaining said pigment from a solvent, e.g. by evaporation of the solvent. In one embodiment, said pigment is obtained by dissolving said pigment in nutritional oil, and is optionally subsequently processed in to capsules for pharmaceutical or nutritional administration. In one embodiment, said pigment is obtained by dissolving said pigment in a solvent or solvent mixture comprising one or more of an organic solvent, a water-based solution, a plant oil, and/or a deep eutectic solvent.

The term "Chlorophyta", as used herein, relates to a division of green algae.

The term "Chlorophyceae", as used herein, relates to a class of green algae which typically have a characteristic arrangement of flagella.

The term *"Haematococcus pluvialis"*, or *"H. pluvialis"*, as used herein, relates to a freshwater species of Chlorophyta which belong to the class of Chlorophyceae. *H. pluvialis* typically produce astaxanthin, and astaxanthin is accumulated in their cyst stage under unfavorable environmental conditions, such as high light intensity, low availability of nutrients, or high salinity. In one embodiment, said unfavorable environmental conditions depend on the biomass concentration, nutrient supply, mixing, aeration, and distance of the light source to the reactor. In one embodiment, said high light intensity refers to a photon flux density of from 50 to 10.000 $\mu$mol m$^{-2}$s$^{-1}$. In one embodiment, low availability of nutrients may relate to a concentration as low as 0 mM nitrate and/or 0 mM phosphate. In one embodiment, high salinity refers to a concentration of up to 10 wt% NaCl. *H. pluvialis* may be present in one of three cell forms; firstly, a motile, biflagellate stage, secondly, a non-motile, naked palmella stage, and thirdly, a non-motile, thick-walled aplanospore stage (also referred to as cyst stage). Astaxanthin is typically accumulated in droplets in the perinuclear cytoplasm in the cyst stage.

The term "solvent", as used herein, relates to a substance which dissolves a solute, such as astaxanthin, resulting in a solution. There are polar and non-polar solvents. Solvents such as methyl-tert-butyl ether, ethyl acetate, butan-1-ol, dichloromethane, chloroform, diethyl ether, ethyl methyl ether, toluene, benzene, ketone, 1,1-dichloroethane, cyclohexane, isopropyl acetate, 2-methyltetrahydofuran, methyl ethyl ketone, methylcyclohexane, 2,2,4-trimethylpentane, xylene, pentan-i-ol, dodecane, decane, acetone, ethanol, propan-2-ol, propan-1-ol, methanol, tetrahydrofuran, tert-butanol, acetonitrile, dimethyl sulfoxide, acetic acid, ethylene glycol, n-alkanes such as n-heptane, and an oil such as nutritional oil, or combinations thereof, can be used in a method of the present invention. In one embodiment, a solvent used in a method of the present invention is a solvent mixture comprising two or more solvents. In one embodiment, in a method of extracting a pigment from microalgae according to the present invention, one solvent or a mixture of solvents may be used to extract said pigment. In one embodiment, the term "solvent" may also relate to a hydrophobic deep eutectic solvent. In one embodiment, a hydrophobic deep eutectic solvent is produced by mixing a hydrogen bond donor and a hydrogen bond acceptor. The term "solvent", as used herein, does not relate to an ionic liquid. In one embodiment, when said pigment is extracted from said flagellated microalgae, wherein said flagellated microalgae are not disrupted, said solvent is selected from methyl-tert-butyl ether, ethyl acetate, butan-1-ol, dichloromethane, chloroform, diethyl ether, ethyl methyl ether, toluene, benzene, ketone, 1,1-dichloroethane, cyclohexane, isopropyl acetate, 2-methyltetrahydofuran, methyl ethyl ketone, methylcyclohexane, 2,2,4-trimethylpentane, xylene, pentan-i-ol, dodecane, decane, acetone, ethanol, propan-2-ol, propan-1-ol, methanol, tetrahydrofuran, tert-butanol, acetonitrile, dimethyl sulfoxide, acetic acid, and ethylene glycol, or a combination thereof, preferably selected from ethyl acetate and methyl-tert-butyl ether. In one embodiment, when said pigment is extracted from disrupted microalgae, said solvent is selected from methyl-tert-butyl ether, ethyl acetate, butan-1-ol, dichloromethane, chloroform, diethyl ether, ethyl methyl ether, toluene, benzene, ketone, 1,1-dichloroethane, cyclohexane, isopropyl acetate, 2-methyltetrahydofuran, methyl ethyl ketone, methylcyclohexane, 2,2,4-trimethylpentane, xylene, pentan-i-ol, dodecane, decane, acetone, ethanol, propan-2-ol, propan-1-ol, methanol, tetrahydrofuran, tert-butanol, acetonitrile, dimethyl sulfoxide, acetic acid, ethylene glycol, n-alkanes, and oil such as nutritional oil. In one embodiment, said solvent is a mixture of solvents, preferably a mixture of ethyl acetate, butan-1-ol, and isopropyl acetate, or a mixture of a nutritional oil with any of ethyl acetate, ethanol, and methanol, wherein said nutritional oil is preferably saturated with any of ethyl acetate, ethanol, and methanol.

The term "stationary phase", as used herein, relates to an immobilized solid or liquid substance within the chromatographic system that allows for separation of a sample. In one embodiment, said stationary phase is preferably an immobilized liquid substance within the chromatographic system.

The term "mobile phase", as used herein, relates to a liquid which dissolves and/or carries sample compounds within a chromatographic system allowing for interaction of said sample compounds with the stationary phase. In one embodiment, the mobile phase contains a pigment which is to be extracted. In one embodiment, the mobile phase contains pigment-enriched microalgae, wherein said microalgae may be present in said mobile phase being intact in a flagellated stage, and/or disrupted in a cyst stage and/or disrupted in a flagellated stage. In one embodiment, said pigment contained in the mobile phase is present intracellularly in said microalgae or extracellularly in cell culture medium.

The term "vapor pressure", as used herein, relates to an indicator of the volatility of a substance and is defined by the pressure exerted by a vapor in a thermodynamic equilibrium with its condensed phases at a given temperature in a closed system. A substance having a high vapor pressure is commonly referred to as volatile. In one embodiment, said vapor pressure relates to a given vapor pressure at room temperature and ambient pressure. The term "high vapor pressure", as used in this context, refers to a vapor pressure of at least 10 mbar. In one embodiment, high vapor pressure relates to at least 64 mbar (such as of heptane), at least 124 mbar (such as of ethyl acetate), or at least 333 mbar (such as of methyl-tert-butyl ether) at 25 °C at ambient pressure. In one preferred embodiment, a solvent used in a method of the present invention has a high vapor pressure.

The term "oil", as used herein, relates to any nonpolar chemical substance that is a viscous liquid at ambient temperatures and is both hydrophobic and lipophilic. An oil may derive from an animal source, a vegetable source, a source from other organisms, or a petrochemical source. In one embodiment, when astaxanthin is extracted from disrupted cells, an oil is used as solvent. In one embodiment, when astaxanthin is extracted from disrupted cells, an edible vegetable or animal oil is used as a solvent, such as olive oil, maize oil, or sunflower oil. In one embodiment, an oil such as a nutritional oil is used as a solvent for said pigment resulting in a final product being an oil comprising a pigment.

**BRIEF DESCRIPTION OF THE FIGURES**

The present invention is now further described by reference to the following figures.

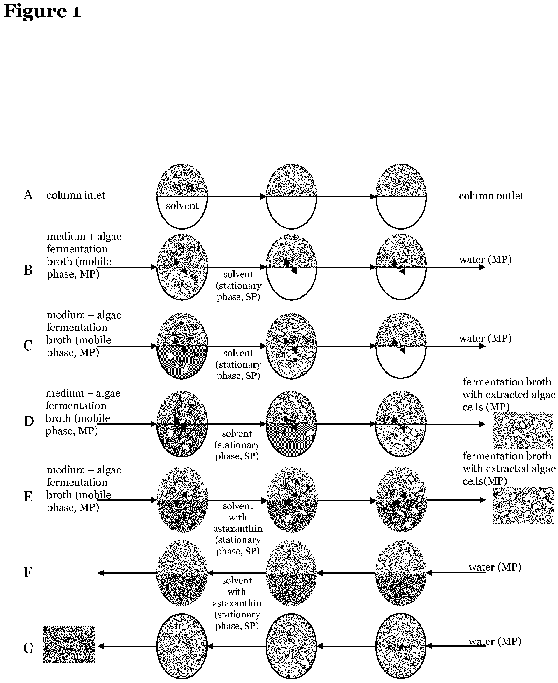

**Figure 1** is a schematic representation of extracting a substance according to the present invention using a CPC system.

In the beginning of the astaxanthin-extraction, the CPC system is filled with a solvent or solvent mixture and the rotation is started. Subsequently, water is pumped into the CPC system to act as mobile phase, and a fraction of the solvent i.e. the stationary phase is replaced by said mobile phase within the CPC system, wherein said replacement depends on the flow rate of the pump and the rotation speed (Figure 1A).

Fermentation broth is subsequently pumped into the CPC system. Mass transfer occurs within the CPC system, so that astaxanthin is extracted from microalgae in flagellated stage into the solvent (Figure 1B).

Alternatively, in one embodiment, said fermentation broth can contain disrupted cells, so that astaxanthin is extracted from said fermentation broth which contains astaxanthin released by disrupted cells. Alternatively, in one embodiment, said fermentation broth can contain both intact cells in flagellated stage and disrupted cells, so that astaxanthin is extracted both from intact flagellated cells and the fermentation broth containing astaxanthin released by disrupted cells.

The fermentation broth is pumped into the CPC system, until the solvent is saturated with astaxanthin (Figure 1C-E). The extracted cells exit the CPC system from the last chamber at the outlet (Figure 1D,E).

Once the solvent is saturated with astaxanthin, water is pumped out of the last chamber to collect the saturated solvent from the first chambers (Figure 1F). Astaxanthin-containing solvent is completely pushed out of the CPC system, and thus collected. The solvent can be evaporated to obtain astaxanthin (Figure 1G).

The extraction process can be repeated any number of times.

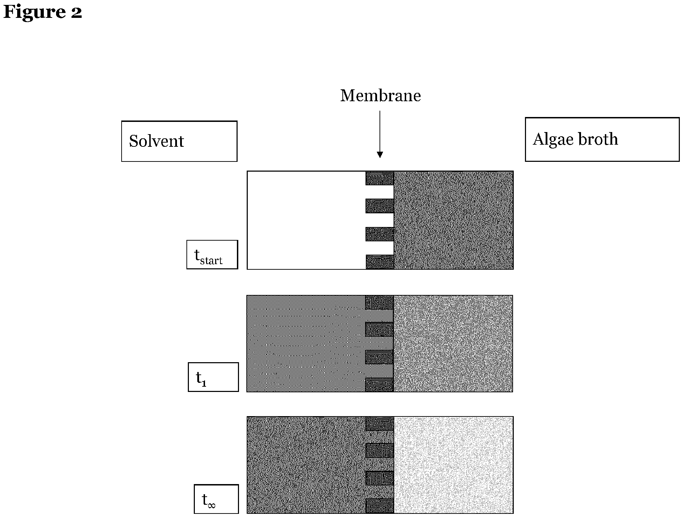

**Figure 2** is a schematic representation of an exemplary membrane-assisted extraction system. The transfer of astaxanthin from the cell broth through the pores of the membrane to the solvent is presented. At time $t_{start}$ an exemplary solvent or solvent mixture is in contact with an astaxanthin containing fermentation broth. As the time progresses, astaxanthin penetrates through the pores and dissolves in the solvent respectively solvent mixture. After an infinite long time $t_\infty$, the astaxanthin distributes between the phases according to its partition coefficient.

In a typical operation, one fluid phase (wetting phase) fills the membrane pores due to capillary forces and the other fluid phase is non-wetting. In one embodiment, a hydrophobic membrane is used. An exemplary solvent (or solvent mixture) is the wetting phase, while the aqueous algae broth is the non-wetting phase. The solvent (or solvent mixture) fills the pores of the membrane. In one embodiment, to stabilize the liquid-liquid interface at the pore mouth, the non-wetting fluid is held at a slightly higher pressure, resulting in a transmembrane pressure of approximately 0.1 bar.

**Figure 3** shows astaxanthin extraction yield from germinating *H. pluvialis* cells using methyl-tert-butyl ether as solvent at 0h, 8h, 16h, 24 h, 32 h, 40h, 48h, 56h and 64 h after inducing the germination. The yield was calculated according to equation 2.

**Figure 4** shows microscope images of partially extracted *H. pluvialis* cells 48 h after the germination was induced. For the extraction 1 ml germinated algae broth was mixed with 5 ml methyl-tert-butyl ether.

**Figure 5** shows astaxanthin concentrations in the extract, 0 h (immediately after inducing the germination) and 24 h after inducing the germination obtained with different solvents (n-heptane, butan-1-ol, ethyl acetate, methyl-tert-butyl ether (MTBE) and dichloromethane).

**Figure 6** depicts shake flask experiments with the solvents butan-1-ol, heptane, methyl-tert-butyl ether, ethyl acetate and dichloromethane (from left to right) at t = 0 h and 24 h after inducing the germination.

**Figure 7** shows the extracted amount of astaxanthin in the 1st collected fraction obtained using operating conditions A, the extracted amount of astaxanthin in the shake flask experiment and the yield $Y_{extract}$ for an injection volume of 0.5 ml and three different elution times 6.9 min, 18.9 min and 36.9 min.

**Figure 8** shows resulting yields $Y_{extract}$ of the three different injection volumes 2 mL ($t_{elution}$ = 8.40 min), 5 mL ($t_{elution}$ = 11.4 min), and 10 mL ($t_{elution}$ = 16.4 min) of operating conditions C. The astaxanthin contents of all the collected fractions and of the injected sample (amount of astaxanthin in the injected fermentation broth) were quantified to calculate the yield ($Y_{extract}$).

**Figure 9** shows the astaxanthin concentration of the injected algae broth of fraction 1, fraction 2, and the remaining fractions of the three different injection volumes 2 mL, 5 mL, and 10 mL of the operating conditions C. With increasing injection volume, an increase of the astaxanthin concentration in the collected fractions 1 and 2 can be seen. The original feed astaxanthin concentration of 65 mg $L^{-1}$ was concentrated to 507 mg $L^{-1}$ and 302 mg $L^{-1}$ in fraction 1 and 2 with an injection volume of 10 mL$^{-1}$.

**Figure 10** shows the principle of membrane-assisted liquid-liquid extraction.

A) Schematic representation of the cross-section of a parallel tube hollow fiber contactor allowing for a non-dispersive contact of two fluids via a microporous membrane. At each pore mouth of the membrane a fluid-fluid interface is formed. The fluid-fluid interface is stabilized by a slight overpressure on one side of the membrane. The membrane serves as a physical separation barrier between the feed and extracting phase (solvent or mixture of solvents). The concentration gradient of the solute in the two phases is the driving force for the mass transfer across fluid-fluid interface and extraction of a solute from one fluid phase (feed) in the other fluid phase (extracting phase).

B) Flow sheet of a pilot plant of a hollow fiber contactor used for the extraction of astaxanthin from algae broth. The algae broth in the right reservoir was pumped in the tubes of the hollow fibers, the solvent in the left reservoir was pumped at the shell side. Both streams flow co-currently and are recirculated.

## EXAMPLES

### *Example 1: Microalgae culture*

*Haematococcus pluvialis* (SAG number 192.80) was procured from the Culture Collection of Algae at the University of Göttingen, Germany (SAG). As culture medium, Bold Modified Basal Freshwater Nutrient Solution (BBM) was used. It was prepared by diluting 20 ml Bold Modified Basal Freshwater Nutrient Solution (50×concentrate) from Sigma-Aldrich (Taufkirchen, Germany), with 980 ml de-ionized water, obtaining the following composition (per liter): 11.42 mg $H_3BO_3$, 25.0 mg $CaCl_2 \cdot 2H_2O$, 0.49 mg $Co(NO_3)_2 \cdot 6H_2O$, 1.57 mg $CuSO_4 \cdot 5H_2O$, 50.0 mg EDTA (free acid), 4.98 mg $FeSO_4 \cdot 7H_2O$, 75 mg $MgSO_4 \cdot 7H_2O$, 1.44 mg $MnCl_2 \cdot 4H_2O$, 0.71 mg $MoO_3$, 0.003 mg $NiCl_2 \cdot 6H_2O$, 31.0 mg KOH, 0.003 mg KI, 175.0 mg $KH_2PO_4$, 75 mg $K_2HPO_4$, 25 mg NaCl, 250.0 mg $NaNO_3$, 0.002 mg $Na_2SeO_3$, 0.001 mg $SnCl_4$, 0.0022 mg $VOSO_4 \cdot 3H_2O$, and 8.82 mg $ZnSO_4 \cdot 7H_2O$. Additionally, 1.64 g of sodium acetate (Molecular biology grade, > 99.0 %) was added to the culture medium. The pH was adjusted to 6.8.

Parts of the *H. pluvialis* colonies were transferred from the agar-plate into a 250 ml Erlenmeyer flask and cultivated in 150 ml BBM + 20 mM sodium acetate. The culture was cultivated at a shaking plate for 16 days at 24 °C until an optical density (OD) of 0.6 at 750 nm was reached. The light was continuously supplied by one cool-fluorescence lamp with a light intensity (photon flux density) of 50 $\mu$mol m$^{-2}$s$^{-1}$. Subsequently, the broth was transferred in into a 2000 ml Erlenmeyer flask, filled up with fresh culture medium (working volume of 1600 ml) and incubated at the previous conditions for 14 days. This broth was used as an inoculum for the cultivation in a self-designed open pond with a total volume of 22 liter. The initial OD at 750 nm was adjusted to 0.1 and using a working volume of 8 liter. Water loss by evaporation was compensated once every 24 h by adding distilled water. The open-pond was illuminated continuously with two cool-fluorescence lamps with a light intensity (photon flux density) of 100 $\mu$mol m$^{-2}$s$^{-1}$ at a constant room temperature of 24±1 °C for 14 days.

The induction of astaxanthin synthesis (enrichment of astaxanthin in the cells) was performed in the open pond at an OD of 0.8 at 750 nm by increasing the light intensity (photon flux density) to 250 $\mu$mol m$^{-2}$s$^{-1}$ for 7 days.

*Example 2: Induction of germination*

To induce germination of *H. pluvialis* cyst cells, 400 ml of the cyst culture broth was transferred into a 500 ml Erlenmeyer flask and placed 24 hours on the shaking plate (175 rpm) at a light intensity (photon flux density) of 50 $\mu$mol m$^{-2}$s$^{-1}$ and a temperature of 24±1 °C. Afterwards the broth was centrifuged at 5500 rpm for 2 min and the supernatant was discarded. The cyst biomass was suspended into fresh BBM + 20 mM sodium acetate and 30 ml were transferred into a 50 ml Erlenmeyer flask with an OD of 4 at 750 nm. Culture conditions at the shaking plate were the same as described in example 1.

*Example 3: Solvent suitability*

Relevant physical properties of several solvents are reported (Table 1). Solvents were chosen regarding their ability to extract astaxanthin from the germinated algae cells, the maximal solubility in water, their hydrophobicity and enthalpy of vaporization.

Table 1: Physical properties of the tested solvents [6].

| | n-heptane | butan-1-ol | methyl-tert-butyl ether | ethyl acetate | dichloromethane |
|---|---|---|---|---|---|
| solubility in water / wt% | 0.00024 (25 °C) | 7.4 (25 °C) | 4.2 (20°C) | 8.08 (25 °C) | 1.73 (25 °C) |
| solubility in water / gl$^{-1}$ | 0.0024 (25 °C) | 80 (25 °C) | 44 (20 °C) | 87.9 (25 °C) | 17.6 (25 °C) |
| log P$_{octanol/water}$ / - | 4.5 | 0.84 | 0.94 | 0.73 | 1.25 |
| boiling point at 1 bar / °C | 98.4 | 117.73 | 55.0 | 77.11 | 40 |
| enthalpy of vaporization $\Delta_{vap}H$, (101.325 kPa, T = 25 °C) / kJ mol$^{-1}$ | 36.57 | 52.35 | 29.82 | 35.60 | 28.82 |

*Example 4: Determination of the biomass concentration*

The dry weight (DW) biomass concentrations were determined in quadruplicates. 1 ml culture aliquot was transferred into 2 ml micro-centrifuge tubes, centrifuged at 5500 rpm for 5 min and the supernatant was discarded. The biomass was washed with distilled water, which was discarded after centrifugation at 5500 rpm and the moist biomass was stored at - 80 °C and freeze dried subsequently. The freeze-dried samples were weighed and the biomass concentration was calculated. Therefore each Eppendorf tube was weighed empty before and with biomass after freeze drying. The dry weight biomass (DW) of each tube was divided through 1 mL.

*Example 5: Astaxanthin quantification*

For the determination of the astaxanthin content in the algae broth, 5 mg of the DW was weighed in with a balance of Satorius (Göttingen, Germany). For cell homogenization the biomass was processed with mortar and pestle. Extraction of the astaxanthin out of the broken cells was achieved by adding 10 ml of dichloromethane. The extraction was repeated three times, until the cell debris was left colorless. The astaxanthin-rich dichloromethane extract was evaporated with a rotary evaporator from Heidolph Instruments (Schwabach, Germany) and saponified for 3 h at room temperature in the dark using the method of Taucher et al. [7]. Therefor 2.25 ml of acetone, 0.25 ml of MeOH and 0.5 ml of 0.05 M NaOH in MeOH were added to the extracted astaxanthin. Afterwards 3 ml petroleum ether were added. The mixture was washed with 3 ml of a 10 wt% aqueous NaCl solution and centrifuged for 2 min at 5500 rpm and the lower phase was discarded. The washing step with the NaCl solution was repeated two more times. The organic phase was evaporated and the extracted astaxanthin was dissolved in 3 ml of solvent B (methanol, MTBE, water, 8:89:3, v/v), which was used in the HPLC method, and filtrated through a 0.22 $\mu$m disposable nylon syringe-filter from Berrytec (Grünwald, Germany).

The de-esterified astaxanthin samples were analyzed with a high-performance liquid chromatography unit (HPLC unit) (LC-20AB prominence Liquid chromatography, Shimadzu, Japan) consisting of an YMC Carotenoid column (C30, 3 $\mu$m, 150 $\times$ 4.6 mm, YMC Co., Japan) and a diode-array detector (SPD-M20A prominence diode array detector, Shimadzu, Japan). As mobile phase, solvent A (methanol, MTBE, water, 81:15:4, v/v) and solvent B (methanol, MTBE, water, 8:89:3, v/v) with the following gradient were used: 2 % solvent B for 11 min, a linear gradient from 2 % solvent B to 40 % solvent B for 7 min, 40 % solvent B for 6.5 min followed by a linear gradient to 100 % solvent B in 2.5 min, 100 % solvent B for 3 min, a linear gradient to 2 % solvent B in 3 min, held for 7 min. The flow rate was 1 ml min$^{-1}$, the injection volume was 10 $\mu$l and the column temperature was kept at 22 °C. For the astaxanthin quantification a calibration curve was created with the chemical standard from Dr. Ehrenstorfer GmbH (Augsburg, Germany). The signal of the diode-array detector was recorded at 478 nm.

### Example 6: Optimum extraction time for germinated H. pluvialis cells

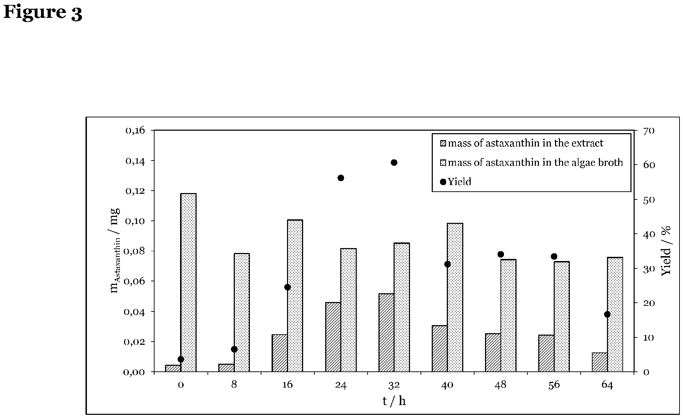

For the determination of the ideal time for the extraction of astaxanthin from the germinated cells , with a time gap of 8 hours two cultures for the germination, culture 1 and culture 2, were prepared as described in the Example 2. For the determination of the astaxanthin yield, one shake flask extraction experiment was performed 0 h, 16 h, 24 h, 40 h, 48 h and 64 h (culture 1) and 8 h, 32 h and 56 h (culture 2) after the germination was initiated. At every of the mentioned time points 1 ml of the algae broth was transferred into a 15 ml falcon tube. 5 ml of methyl-tert-butyl ether were added and the binary mixture was shaken intensively for 30 min with the Multi Bio RS-24 from bioSan (Riga, Latvia) at a room temperature of 24$\pm$1 °C. After that, to separate the phases, the sample was centrifuged for 2 min at 5500 rpm. 4 ml of the astaxanthin-rich methyl-tert-butyl ether phase were withdrawn and evaporated with a rotary vacuum evaporator from Heidolph Instruments (Schwabach, Germany). The astaxanthin content in the extracts was quantified using the HPLC method described in Example 5. The biomass concentration and astaxanthin content of the algae broth for all studied time intervals was quantified as described in Example 4 and Example 5, respectively. The mass of astaxanthin in the extract, in the algae broth and the yield are presented in Figure 3.

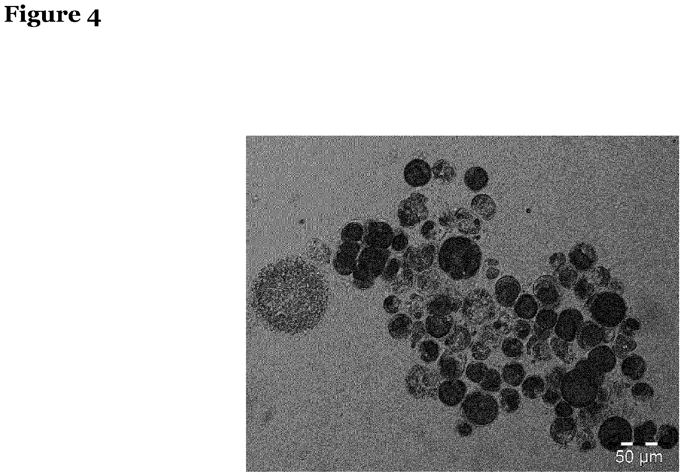

The optimal time for the extraction of astaxanthin from the germinated cells was determined to be between 24 h and 32 h after the germination was induced (Figure 3). The extraction yield, calculated according equation 3, was nearly constant between 24 h and 32 h reaching yields of 56 to 60 % of the total astaxanthin available in the algal broth. After 40 h the yield decreased to 31 % and further to 17 %, 64 h after the germination was induced. The astaxanthin content in the algal broth remained constant within the investigated time range. The decrease of the yield after 32 h was caused by morphological changes of the germinated cells. An increasing number of germinated cells lost their flagella and built up a robust cell structure, with reduced permeability for methyl-tert-butyl ether (Figure 4).

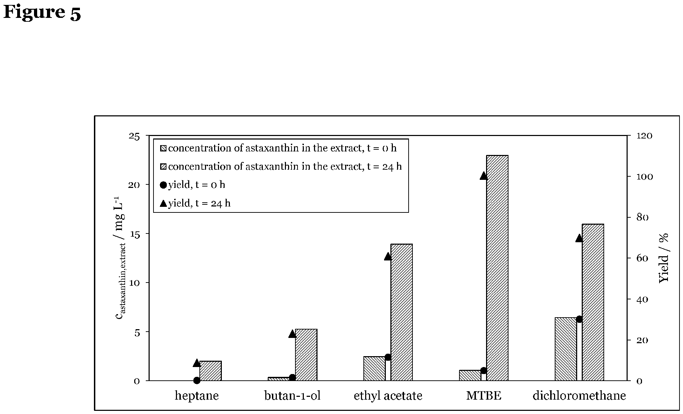

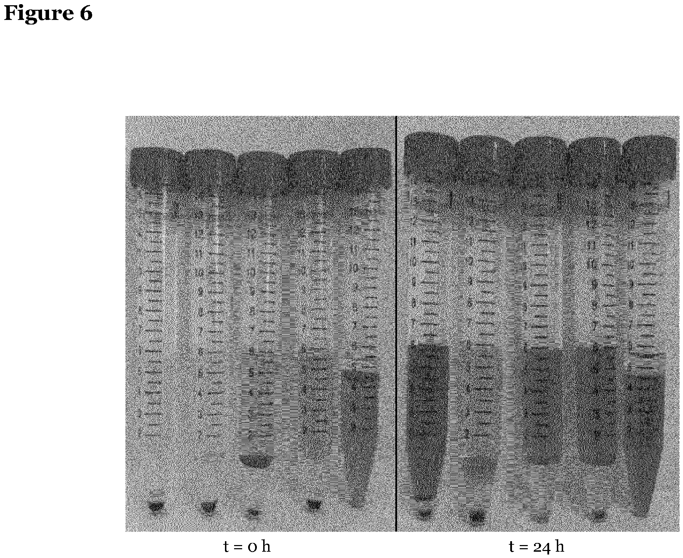

The time points t = 0 h (extraction was performed immediately after adding new medium) and t = 24 h after the induction of the germination were taken to determine the extraction efficiency of the solvents shown in Table 1. Figure 5 shows the astaxanthin concentration in heptane, butan-1-ol, ethyl acetate, methyl-tert-butyl ether (MTBE), and dichloromethane of the extract phase, after mixing 5 ml of each solvent with 1 ml feed at the mentioned times. The extraction efficiency of each solvent is increasing between t = 0 h and t = 24 h, due to the increasing number of germinated algae cells. 24 h after inducing the germination, the extraction efficiencies of the tested solvents was in the order [methyl-tert-butyl ether] > [dichloromethane] > [ethyl acetate] > [butan-i-ol] > [heptane] (Figure 5 and Figure 6).

### Example 7: Extraction with countercurrent chromatography (CCC) and centrifugal partition chromatography (CPC) system

CCC experiments were carried out on a countercurrent chromatography column, model HPCCC-Mini Centrifuge (0.8 mm i.d.) with a $\beta$-value between 0.5 to 0.78 from Dynamic Extractions (Wales) and a column volume of 18.2 ml. Two isocratic Gilson 306 pumps (Gilson, USA), equipped with an 806 Manometric Module (Gilson, USA), were used for delivering the mobile and stationary phases.

CCC extraction experiments were conducted using methyl-tert-butyl ether as an extraction solvent. Therefore, methyl-tert-butyl ether was stirred for two hours with BBM + 20 mM sodium acetate culture medium at a room temperature of 24$\pm$1 °C. The equilibrated system was split into the upper solvent-rich phase and the lower culture medium-rich phase using a separatory funnel. The phases were degassed in an ultrasonic bath. The CCC unit was prepared by filling the column with the solvent-rich phase i.e. the stationary phase. Rotation was set at 1900 rpm and the culture medium, saturated with methyl-tert-butyl ether (the mobile phase) was pumped in the descending mode with a flow rate F of 1 ml min$^{-1}$. After equilibrium conditions in the column were reached, the germinated biomass was injected to the column via an injection loop. After the corresponding elution time (shown in Table 2 for each operating condition), the stationary phase was pushed out of the column. That was done by switching from the descending mode to the ascending mode. The stationary phase was fractionated into 2 mL HPLC vials until no more stationary phase was coming out of the column. Defined parts of the collected fractions were pipetted into round bottom flasks, evaporated and further processed for the HPLC analysis as explained in Example 5.

The CPC experiment was performed in the CPC 250 PRO SPECIAL BIO VERSION column with twin cells, with a total column volume of 250 ml, from Gilson Purifications SAS (formerly Armen Instruments, France):
The CPC column has 12 disks, where each disk contains 20 engraved twin-cells; in total 240 cells. The column was connected to a pressure vessel (Apache Stainless Equipment Corporation, USA) with a total volume of 5 Liter for pumping the algae broth into the CPC. An overpressure of up to 7.3 bar was provided by the in-house compressed air line.
The CPC experiment was carried out, using ethyl acetate as the extraction solvent. Ethyl acetate was stirred for 2 hours with deionized water at $24 \pm 1$ °C. The separated phases were degassed with an ultrasonic bath. The CPC unit was filled with solvent-rich phase i.e. the stationary phase. After the rotational speed of the system had been set to 1350 rpm, the mobile phase (water saturated with ethyl acetate) was fed to the CPC unit at a pressure of 7.3 bar in the descending mode, what results in a flow rate of 30 ml min$^{-1}$ at the set pressure. After that, 720 mL of the germinated algae broth with an OD of 4 at 750 nm was pumped into the CPC column with the same pressure. After 24 min (corresponds to 720 mL algae broth at this flow rate), the flow of algae into the CPC was stopped. The pressure vessel was filled with deionized water (saturated with ethyl acetate) and the stationary phase was pushed out of the column in the ascending mode. The stationary phase was fractionated into 15 mL falcon tubes until no more stationary phase was coming out of the column. Defined parts of the collected fractions were pipetted into round bottom flasks, evaporated and further processed for the HPLC analysis as explained in Example 5.

The elution time $t_{elution}$ is the time span between the start of the injection (i.e. pumping) of the biomass into the CCC (CPC) column and switching from descending to ascending mode to push out the stationary phase from the column. Looking at the injected biomass as a tracer, equation 1 gives the minimum time required for the extracted biomass (cells) to leave the column.

| equation 1: | $t_{elution,min} = \dfrac{V_{MP} + V_{injection}}{F}$ |
|---|---|
| $V_{MP}$ is the volume of the mobile phase and $V_{injection}$ is the injected volume. | |

After the elution time, $t_{elution}$, the stationary phase was pushed out the column in the ascending mode by pumping the culture medium (CCC), respectively water (CPC)-rich phase. In both cases the phase was saturated with the solvent used for the extraction, methyl-tert-butyl ether for CCC and ethyl acetate for CPC. The fractions of the astaxanthin dissolved in the solvent coming out from the column were collected, evaporated and the astaxanthin content determined according to the procedure described in Example 5.

The yield $Y_{feed}$ was calculated as quotient of the mass of astaxanthin in the collected fractions $m_{astaxanthin,fraction}$ to the astaxanthin mass in injected feed biomass $m_{astaxanthin,feed}$, as shown in equation 2.

| equation 2: | $Y_{feed} = \dfrac{m_{astaxanthin,fraction}}{m_{astaxanthin,feed}} \cdot 100$ |
|---|---|

Additionally, at every CCC (CPC) experiment, three shake flask experiments were performed, in order to determine the extractable amount of astaxanthin from the cells in the corresponding cell stage. Therefore 1 ml of the algae broth was mixed with 5 ml methyl-tert-butyl ether (CCC experiments) respectively ethyl acetate (CPC experiment) for 30 min. After centrifugation at 5500 rpm for 5 min, 4 ml of the solvent was taken and the astaxanthin content was determined according to the procedure described in Example 5. A yield $Y_{extract}$, which takes the extractable amount of astaxanthin from the cells in the corresponding cell stage into account, was defined according to equation 3,

| equation 3: | $Y_{extract} = \dfrac{m_{astaxanthin,fraction}}{N \cdot m_{astaxanthin,\ extract,shake\ flask}} \cdot 100$ |
|---|---|

wherein $m_{astaxanthin,extract,shake\ flask}$ is the extracted amount of astaxanthin in the extract of the shake flask experiment. As the shake flask experiment was always performed with 1 ml algae broth, the factor N is needed to adjust the value to the feed injected into the CCC/CPC. For example, if 5 mL algal broth was injected into the CCC/CPE and the shake flask experiment was carried out with 1 mL algae broth, this results in a value of 5 for N.

*Example 8: CCC and CPC experiments*

According to the results shown in the previous example such as example 6, methyl-tert-butyl ether was used as the extraction solvent in all CCC experiments and ethyl acetate in the CPC experiment. The germination was induced according to the conditions described in the previous examples. The following four operating conditions were examined in the CCC and one in the CPC column (Table 2). The obtained results are summarized in Table 2.

Table 2: Operating conditions for the experiments A - D in the CCC column and E in the CPC column.

| | A | | | B | | | | | | C | | | D | | E |
|---|---|---|---|---|---|---|---|---|---|---|---|---|---|---|---|
| $C_{\text{biomass injected}}$ / mg mL$^{-1}$ | 4.95 | | | 2.95 | | | 2.95 | | | 4.97 | | | 16.8 | 6.72 | 8.65 |
| $V_{\text{inj}}$ / mL | 0.5 | | | 0.5 | | | 2 | | | 2 | 5 | 10 | 2 | 5 | 720 |
| $m_{\text{biomass injected}}$ / mg | 2.5 | | | 1.48 | | | 5.9 | | | 10.0 | 24.9 | 49.8 | 33.6 | | 6055 |
| $m_{\text{astaxathin injected}}$ / mg | 0.026 | | | 0.027 | | | 0.108 | | | 0.1 | 0.33 | 0.66 | 0.57 | | 63.95 |
| $t_{\text{elution}}$ / min | 6.9 | 18.9 | 36.9 | 6.9 | 18.9 | 36.9 | 8.4 | 20.4 | 38.4 | 8.4 | 11.4 | 16.4 | 8.4 | 11.4 | 24 |
| $C_{\text{1st fraction}}$ / mg$_{\text{astaxanthin}}$ L$^{-1}$ | 2.5 | 2.5 | 2.5 | 4.9 | 5.6 | 5.0 | 29.5 | 29.8 | 25.5 | 46.9 | 124.8 | 507.6 | 195.7 | 179.6 | 338.8 |
| $Y_{\text{extract}}$ / % | 61.3 | 76.1 | 58.9 | 47.7 | 47.7 | 48.6 | 48.4 | 72.0 | 53.7 | 113.8 | 129.1 | 113.1 | 44.2 | 44.9 | 21.9 |
| $Y_{\text{feed}}$ / % | 11.1 | 13.8 | 10.7 | 27.5 | 27.5 | 28.0 | 27.9 | 41.5 | 31.0 | 65.9 | 74.7 | 65.5 | 39.1 | 39.1 | 6.4 |

• Operating conditions A

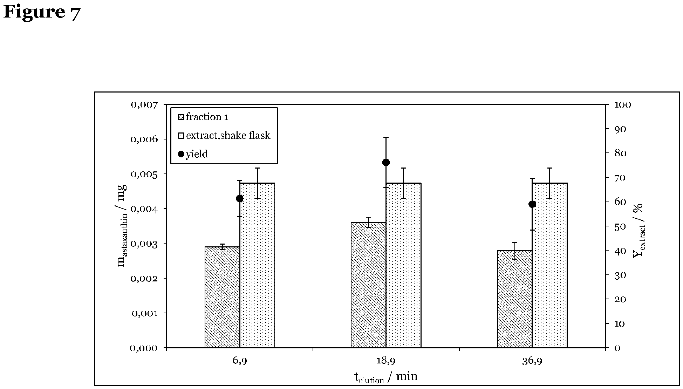

In the operating conditions A, three different elution times were examined, namely 6.9 min, 18.9 min and 36.9 min. The injection volume (germinated algae broth) was 0.5 ml resulting in an injected amount of astaxanthin of 0.026 mg. Here, the influence of increasing elution times on the yield was investigated. Only the first and most concentrated fraction was analyzed for its astaxanthin content and used for the calculation of the yield. As presented in Figure 7, an increase of $t_{elution}$ from 6.9 min to 36.9 min did not affect the yield $Y_{extract}$ of extracted astaxanthin. The calculated yields $Y_{extract}$ were in the range of 60 to 76 %.

• Operating conditions B

To verify the results from operating condition A, in operating condition B three different elution times were examined for the injection volumes of 0.5 mL and 2 mL. For 0.5 mL the elution times were chosen similar to operating conditions A, were the column was emptied 6.9, 18.9 and 36.9 min after the injection of 0.5 mL germinated algae cells. For the injection volume of 2 mL of biomass, the elution times 8.4 min, 20.4 min and 38.4 min were examined. Only the first and most concentrated fraction was analyzed for its astaxanthin content and used for the calculation of the yield.
Similar to the results of operating conditions A, the increase of the elution time did not affect the yields $Y_{extract}$ and $Y_{feed}$. For the injection of 0.5 mL, the values obtained for $Y_{extract}$ were around 48 %, values for $Y_{feed}$ were around 28 %. The injections of 2 mL germinated algae broth show a similar trend. For an elution time of 8.4 min and 38.4 min, $Y_{extract}$ is 48 % and 54 %. For an elution time of 20.4 min, $Y_{extract}$ was calculated to be 72 %. For $Y_{feed}$, similar trends can be seen, yields of 27 %, 42 % and 31 % were obtained for the elution times of 8.4 min, 20.4 min and 36.4 min, respectively.

• Operating conditions C

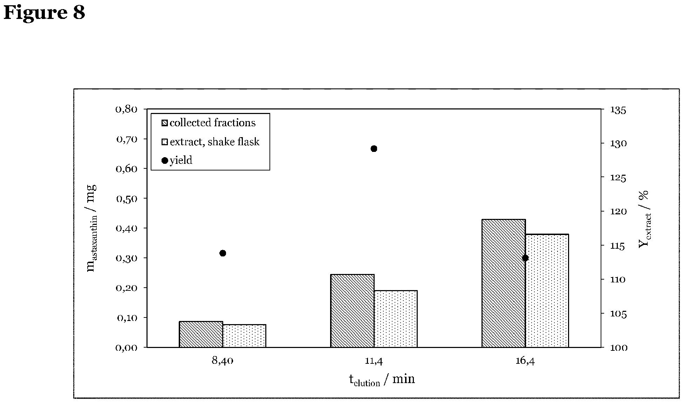

In the operating conditions C, the influence of three different injection volumes, 2 mL, 5 mL and 10 mL on the yield and concentration of astaxanthin was examined. The astaxanthin content in the first two collected fractions was determined and the content of the low concentrated remaining fractions was determined after pouring these fractions together. For the calculation of the yields with equation (2) and (3), the mass of astaxanthin ($m_{astaxanthin,fraction}$) in fraction 1, fraction 2 and in the remaining fractions were summed up. As shown in the previous operating conditions A and B, an elution time longer than calculated with equation (1) doesn't affect the extracted amount of astaxanthin respectively the achieved yields. Consequently, $t_{elution}$ were determined to be 8.4 min, 11.4 min and 16.4 min according to equation (1) for the injected volumes of 2 mL, 5 mL and 10 mL. The calculated yields $Y_{extract}$ were 113 % for the elution times of 8.4 min and 16.4 min and 130 % for 11.4 min and are presented in Figure 8. The achieved yields $Y_{feed}$ were 65 % for the elution times of 8.4 min and 16.4 min and 75 % for an elution time of 11.4 min.

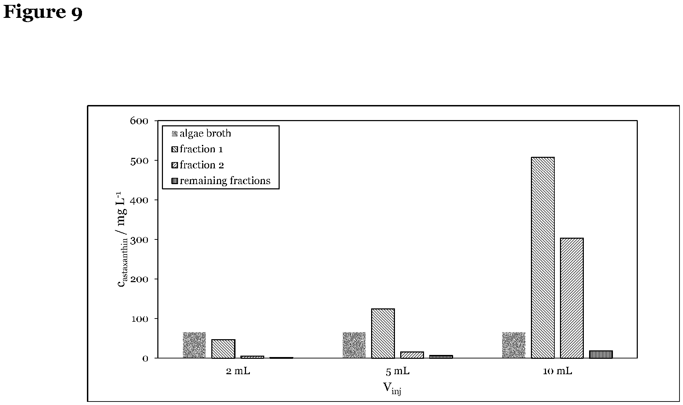

In Figure 9 the concentrations of the injected algae broth and the collected fraction 1, fraction 2 and remaining fractions of the three injection volumes are shown. As it can be seen, the starting concentration of 65 $mg_{astaxanthin}$ L$^{-1}$ in the injected algal broth is concentrated to 500 $mg_{astaxanthin}$ L$^{-1}$ and 300 $mg_{astaxanthin}$ L$^{-1}$ in fraction 1 and fraction 2 when 10 mL were injected.

• Operating conditions D

In operating conditions D, two different injection volumes of the germinated algae broth, 2 mL and 5 mL, were injected with two different biomass concentrations. For an injection volume of 2 mL, 16.8 mg mL$^{-1}$ biomass and for an injection volume of 5 mL, 6.72 mg mL$^{-1}$ biomass were injected. Thus, the injected amount of biomass respectively astaxanthin was the same in these experiments. The achieved $Y_{extract}$ was 44 %, $Y_{feed}$ was 39 % for the injected volumes. In the run, where 2 mL algae broth were injected, the concentrations reached in the first and second fraction were 195 $mg_{astaxanthin}$ L$^{-1}$ and 48 $mg_{astaxanthin}$ L$^{-1}$. Similar values were reached, when 5 mL were injected resulting in concentrations of 180 $mg_{astaxanthin}$ L$^{-1}$ and 50 $mg_{astaxanthin}$ L$^{-1}$ for fraction 1 and 2.

• Operating conditions E

In the CPC run, 720 ml germinated cell broth was injected into the CPC, corresponding to a biomass of 6055 mg and 63.95 mg astaxanthin, respectively. In the first collected fraction an astaxanthin content of 338 mg L$^{-1}$ was measured. The yield $Y_{extract}$ was 21.9%.

***Example 9: Membrane-assisted liquid-liquid extraction***

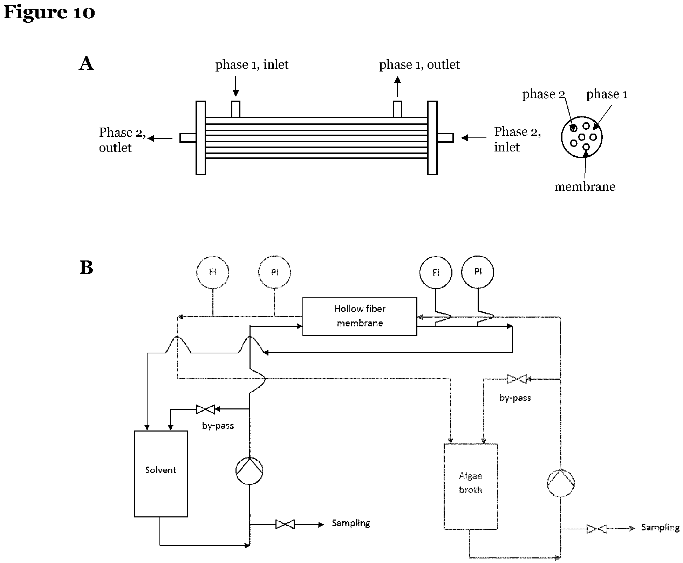

A pilot plant with PTFE hollow fibers with a total surface area of 0.1619 m$^2$ was used for membrane-assisted liquid-liquid extraction. The solvent ethyl acetate and the homogenized *H. pluvialis* cysts were placed in two separate reservoirs. The solvent ethyl acetate was pumped in to the shell side and homogenized *H. pluvialis* cysts were pumped in the lumen side (in the tubes) of the hollow fiber membrane module. The two streams, ethyl acetate and the homogenized *H. pluvialis* cysts broth were pumped concurrently and circulated at the respective site of the membrane (Fig 10). The run was stopped after 3 h. Astaxanthin was extracted from the fermentation broth into the solvent. Before the run, a shake flask experiment was performed similar to the CCC/CPC experiments. The absorbance of the extracted pigments was measured with UV/Vis spectroscopy at 478 nm to be 23.5. Three hours after starting the run, an absorbance of 0.333 at 478 nm was measured for the solvent circulated in the shell side of the plant. Assuming an infinite long extraction time, the same yield as in a shake flask experiment i.e. around 90%, this means a one stage extraction, can be expected.

## REFERENCES

[1] Koller M, Muhr A, and Braunegg G. Microalgae as versatile cellular factories for valued products. Algal Research, 2014; 6, 52-63.

[2] Shah MMR, Liang Y, Cheng JJ, Daroch M. Astaxanthin-Producing Green Microalga Haematococcus pluvialis: From Single Cell to High Value Commerical Products. Frontiers in Plant Science. 2016; 7.

[3] R. Praveenkumar, K. Lee, J. Lee and Y.-K. Oh, Breaking dormancy: an energy-efficient means of recovering astaxanthin from microalgae, Green Chemistry, 2014; 17, 1226-1234.

[4] Marchal L, Mojaat-Guemir M, Foucault A and Pruvost J. Centrifugal partition extraction of β-carotene from Dunaliella salina for efficient and biocompatible recovery of metabolites, Bioresource Technology, 2013; 134, 396-400.

[5] Xiping Du, Congcong Dong, Kai Wang, Zedong Jiang, Yanhong Chen,Yuanfan Yang, Feng Chen, Hui Ni. Separation and purification of astaxanthin from Phaffia rhodozyma by preparative high-speed counter-current chromatography. Journal of Chromatography B. 2016; 1029-1030,191-197.

[6] David R. Lide e. CRC Handbook of Chemistry and Physics, Internet Version 2005. 2005.

[7] Taucher J, Baer S, Schwerna P, Hofmann D, Hümmer M, Buchholz R, et al. Cell Disruption and Pressurized Liquid Extraction of Carotenoids from Microalgae. Journal of Thermodynamics & Catalysis. 2016; 7(1):7.

The features of the present invention disclosed in the specification, the claims, and/or in the accompanying figures may, both separately and in any combination thereof, be material for realizing the invention in various forms thereof.

## Claims

1. A method of extracting a pigment from microalgae, comprising the following steps:

    a) Providing microalgae in an aqueous culture medium, wherein said microalgae are enriched with a pigment,
    b) Inducing said microalgae to enter a flagellated stage using a germination-inducing condition, and/or disrupting said microalgae resulting in a suspension comprising said disrupted microalgae and said pigment,
    c) Extracting said pigment from said flagellated microalgae and/or from said suspension using a liquid-liquid extraction system comprising a solvent, wherein the liquid-liquid extraction system is selected from a counter-current chromatography system, a centrifugal partition chromatography system, and a membrane-assisted liquid-liquid extraction system.

2. The method according claim 1, wherein said method comprises the following steps:

    a) Providing microalgae in an aqueous culture medium, wherein said microalgae are enriched with a pigment,

b) Inducing said microalgae to enter a flagellated stage using a germination-inducing condition,

c) Extracting said pigment from said flagellated microalgae using a liquid-liquid extraction system comprising a solvent, wherein the liquid-liquid extraction system is selected from a countercurrent chromatography system, a centrifugal partition chromatography system, and a membrane-assisted liquid-liquid extraction system.

3. The method according to claim 1, wherein said method comprises the following steps:

a) Providing microalgae in an aqueous culture medium, wherein said microalgae are enriched with a pigment,

b) Disrupting said microalgae resulting in a suspension of said disrupted microalgae and said pigment,

c) Extracting said pigment from said suspension using a liquid-liquid extraction system comprising a solvent, wherein the liquid-liquid extraction system is selected from a countercurrent chromatography system, a centrifugal partition chromatography system, and a membrane-assisted liquid-liquid extraction system.

4. The method according to claim 1 or 2, wherein said microalgae are initially in a cyst stage and are induced to enter a flagellated stage by means of a germination-inducing condition.

5. The method according to claim 1, 2, or 4, wherein said germination-inducing condition is selected from a phototrophic condition, a mixotrophic condition, and a heterotrophic condition.

6. The method according to claim 1 or 3, wherein said microalgae are disrupted mechanically, for example by using a homogenizer, a grinder, a bead mill, beadbeating, a blender, sonication, pressure cycling, a microfluidizer, an expeller press, or freezing and thawing cycles.

7. The method according to any of the foregoing claims, wherein said step c) is followed by
step d) obtaining said pigment by lyophilization, freezing, vaporization of said solvent, or by dissolving said pigment in a nutritional oil, or another solvent, such as an organic solvent, a water based solution, a plant oil, or a deep eutectic solvent, or a combination thereof.

8. The method according to any of the foregoing claims, wherein said microalgae have become enriched with said pigment by nutrient depletion, excessive light exposure, high salinity, and/or overexpression resulting from genetic modification of said microalgae.

9. The method according to any of the foregoing claims, wherein said microalgae are Chlorophyta, preferably Chlorophyceae, more preferably *Haematococcus pluvialis.*

10. The method according to any of claims 1-8, wherein said microalgae are selected from *Haematococcus pluvialis, Chlorella zofingiensis, Neochloris wimmeri,* and *Chlamydomonas nivalis.*

11. The method according to any of the foregoing claims, wherein said liquid-liquid extraction system is a membrane-assisted liquid-liquid extraction system.

12. The method according to any of claims 1-10, wherein said liquid-liquid extraction system is a liquid-liquid chromatography system selected from a centrifugal partition chromatography system and a countercurrent chromatography system.

13. The method according to any of the foregoing claims, wherein said solvent has a vapor pressure of at least 10 mbar, preferably of at least 64 mbar, at 25 °C and ambient pressure.

14. The method according to any of the foregoing claims, wherein said solvent is selected from methyl-tert-butyl ether, ethyl acetate, butan-1-ol, dichloromethane, chloroform, diethyl ether, ethyl methyl ether, toluene, benzene, ketone, 1,1-dichloroethane, cyclohexane, isopropyl acetate, 2-methyltetrahydofuran, methyl ethyl ketone, methylcyclohexane, 2,2,4-trimethylpentane, xylene, pentan-i-ol, dodecane, decane, acetone, ethanol, propan-2-ol, propan-1-ol, methanol, tetrahydrofuran, tert-butanol, acetonitrile, dimethyl sulfoxide, acetic acid, ethylene glycol, n-alkanes, and oil such as nutritional oil, or a combination thereof, preferably selected from ethyl acetate and methyl-tert-butyl ether, or a combination thereof.

15. The method according to any of the foregoing claims, wherein said pigment is a keto-carotenoid, preferably astaxanthin.

# Figure 1

**Figure 2**

**Figure 3**

Figure 4

## Figure 5

**Figure 6**

t = 0 h                                    t = 24 h

**Figure 7**

**Figure 8**

**Figure 9**

Figure 10

A

phase 1, inlet    phase 1, outlet

phase 2    phase 1

Phase 2,
outlet

Phase 2,
inlet

membrane

B

Hollow fiber
membrane

by-pass

by-pass

Solvent

Algae
broth

Sampling

Sampling

Europäisches
Patentamt

European
Patent Office

Office européen
des brevets

## EUROPEAN SEARCH REPORT

Application Number

EP 18 20 4120

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI H-B ET AL: "Preparative isolation and purification of astaxanthin from the microalga Chlorococcum sp. by high-speed counter-current chromatography", JOURNAL OF CHROMATOGRAPHY, ELSEVIER, AMSTERDAM, NL, vol. 925, no. 1-2, 3 August 2001 (2001-08-03), pages 133-137, XP004296306, ISSN: 0021-9673, DOI: 10.1016/S0021-9673(01)01022-6 * paragraphs [02.3], [0003]; figure 2; table 1 * | 1,3, 6-10, 12-15 | INV. C12P23/00 B01D11/00 G01N30/42 |
| X | WO 2014/134411 A1 (VALICOR INC [US]) 4 September 2014 (2014-09-04) * [00021],[00026],[00040],[00070]-[00083] * | 1,3,9, 10,13-15 | |
| X | LUC MARCHAL ET AL: "Centrifugal partition extraction of [beta]-carotene from Dunaliella salina for efficient and biocompatible recovery of metabolites", BIORESOURCE TECHNOLOGY, vol. 134, 13 February 2013 (2013-02-13), pages 396-400, XP055541111, AMSTERDAM, NL ISSN: 0960-8524, DOI: 10.1016/j.biortech.2013.02.019 * 2.2,2.5.2,3.2 * | 1,2,8,9, 12-14 | TECHNICAL FIELDS SEARCHED (IPC) C12P G01N B01D |

-/--

The present search report has been drawn up for all claims

| Place of search | Date of completion of the search | Examiner |
|---|---|---|
| Berlin | 11 January 2019 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 20 4120

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JIAN-PING YUAN ET AL: "Hydrolysis Kinetics of Astaxanthin Esters and Stability of Astaxanthin of Haematococcus pluvialis during Saponification", JOURNAL OF AGRICULTURAL AND FOOD CHEMISTRY, vol. 47, no. 1, 1 January 1999 (1999-01-01), pages 31-35, XP055540265, US ISSN: 0021-8561, DOI: 10.1021/jf980465x * page 31, column 2, paragraph 3 - page 32, column 1, paragraph 1 * | 1,3, 6-10, 12-15 | |
| A | KAY GRÜNEWALD ET AL: "Secondary carotenoid accumulation in flagellates of the green alga Haematococcus lacustris", EUROPEAN JOURNAL OF PHYCOLOGY, vol. 32, no. 4, 26 November 1997 (1997-11-26), pages 387-392, XP055540359, DE ISSN: 0967-0262, DOI: 10.1080/09670269710001737329 * page 387, column 2, paragraph 3 - page 388, column 1, paragraph 3 * | 1,2,4,5, 8,9 | |
| A | SHENGZHAO DONG ET AL: "Four Different Methods Comparison for Extraction of Astaxanthin from Green Alga Haematococcus pluvialis", THE SCIENTIFIC WORLD JOURNAL, vol. 2014, 1 January 2014 (2014-01-01), pages 1-7, XP055539500, ISSN: 2356-6140, DOI: 10.1155/2014/694305 | 1-15 | TECHNICAL FIELDS SEARCHED (IPC) |

The present search report has been drawn up for all claims

| Place of search | Date of completion of the search | Examiner |
|---|---|---|
| Berlin | 11 January 2019 | Schönwasser, D |

EPO FORM 1503 03.82 (P04C01)

page 2 of 2

## ANNEX TO THE EUROPEAN SEARCH REPORT
## ON EUROPEAN PATENT APPLICATION NO.

EP 18 20 4120

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2019

| Patent document cited in search report | | Publication date | Patent family member(s) | | Publication date |
|---|---|---|---|---|---|
| WO 2014134411 | A1 | 04-09-2014 | US | 2014243540 A1 | 28-08-2014 |
| | | | WO | 2014134411 A1 | 04-09-2014 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KOLLER M ; MUHR A ; BRAUNEGG G.** Microalgae as versatile cellular factories for valued products. *Algal Research,* 2014, vol. 6, 52-63
- **SHAH MMR ; LIANG Y ; CHENG JJ ; DAROCH M.** Astaxanthin-Producing Green Microalga Haematococcus pluvialis: From Single Cell to High Value Commerical Products. *Frontiers in Plant Science,* vol. 2016, 7
- **R. PRAVEENKUMAR ; K. LEE ; J. LEE ; Y.-K. OH.** Breaking dormancy: an energy-efficient means of recovering astaxanthin from microalgae. *Green Chemistry,* 2014, vol. 17, 1226-1234
- **MARCHAL L ; MOJAAT-GUEMIR M ; FOUCAULT A ; PRUVOST J.** Centrifugal partition extraction of β-carotene from Dunaliella salina for efficient and biocompatible recovery of metabolites. *Bioresource Technology,* 2013, vol. 134, 396-400

- **XIPING DU ; CONGCONG DONG ; KAI WANG ; ZEDONG JIANG ; YANHONG CHEN ; YUANFAN YANG ; FENG CHEN ; HUI NI.** Separation and purification of astaxanthin from Phaffia rhodozyma by preparative high-speed counter-current chromatography. *Journal of Chromatography B.,* 2016, vol. 1029 (1030), 191-197
- **DAVID R. ; LIDE E.** CRC Handbook of Chemistry and Physics, Internet Version 2005. 2005
- **TAUCHER J ; BAER S ; SCHWERNA P ; HOFMANN D ; HÜMMER M ; BUCHHOLZ R et al.** Cell Disruption and Pressurized Liquid Extraction of Carotenoids from Microalgae. *Journal of Thermodynamics & Catalysis,* 2016, vol. 7 (1), 7